# EUROPEAN PATENT SPECIFICATION

(11) **EP 3 155 395 B1**
(45) Date of publication and mention of the grant of the patent: **02.06.2021**
(21) Application number: 15806086.3
(22) Date of filing: 09.06.2015
(51) Int. Cl.: C12Q 1/6806, C12N 5/078, C12N 15/10

(54) **METHODS AND COMPOSITIONS FOR STABILIZATION OF NUCLEIC ACIDS IN A BLOOD SAMPLE AT AMBIENT TEMPERATURES**
VERFAHREN UND ZUSAMMENSETZUNGEN ZUR STABILISIERUNG VON NUKLEINSÄUREN IN EINER BLUTPROBE BEI UMGEBUNGSTEMPERATUR
PROCÉDÉ ET COMPOSITION POUR LE STABILISATION D'ACIDES NUCLÉIQUES DANS UN ÉCHANTILLON DE SANG À DES TEMPÉRATURES AMBIANTES

(30) Priority: 10.06.2014 US 201462010164 P
(43) Date of publication of application: 19.04.2017
(73) Proprietor: Biomatrica, Inc., San Diego, CA 92121 (US)
(72) Inventor: MULLER, Rolf, Del Mar, CA 92014 (US); WHITNEY, Scott, San Diego, CA 92126 (US); DIAZ, Paul, Riverside, CA 92505 (US)
(74) Representative: Glawe, Delfs, Moll
(86) International application number: PCT/US2015/034968
(87) International publication number: WO 2015/191633

(56) References cited:
- WO-A1-2008/108549
- WO-A1-2008/111981
- WO-A1-2013/045458
- WO-A1-2014/029791
- US-A- 4 473 552
- US-A1- 2005 124 965
- US-A1- 2009 259 023
- US-A1- 2010 178 210
- US-A1- 2010 209 930
- US-A1- 2013 209 997
- US-A1- 2014 080 112
- PE TERS ET AL.: 'Sensitivity of Human, Munne, and Rat Cells to 5-Fluorouracil and 5'-Deoxy-5- fluorouridine in Relation to Drug-metabolizing Enzymes' CANCER RESEARCH vol. 46, January 1986, pages 20 - 28, XP055243439

## Description

### CROSS-REFERENCE

This application claims the benefit of U.S. Provisional Application No. 62/010,164, filed June 10, 2014.

### BACKGROUND OF THE INVENTION

### 1. Technical Field

The present invention relates generally to stabilization of one or more non-denatured nucleic acid molecule and/or polypeptide, or exosome in a blood sample at ambient temperatures. In particular, the invention relates to formulations, compositions, articles of manufacture, kits and methods for substantially stable storage of one or more non-denatured nucleic acid molecule and/or polypeptide, or exosome in a blood sample at ambient temperatures.

### 2. Background

Compositions comprising a pH buffer for substantially storage of nucleic acids and polypeptides in biological samples without refrigeration and without the use of formaldehyde donors are known from US 2013/209997 A1. Similar compositions comprising EDTA as a key component are known from WO 2013/045458 A1 and WO 2008/111981 A1, while the formulation of the invention as defined in the enclosed claims does not comprise such a chelating agent. Whole blood is a complex mixture of cells, nucleic acids, proteins and various other analytes. In particular, blood components include, but are not limited to: cells, such as leukocytes (monocytes, lymphocytes and granulocytes), erythrocytes, thrombocytes and circulating tumor cells; nucleic acid molecules, such a circulating-free DNA (cfDNA); polypeptides, such as lipoproteins, albumin and serum proteins, and other various analytes.

A constant need for blood donors exists for whole blood, in part, due to the relatively short half-life and storage requirements for whole blood. For instance, whole blood collected for transfusion must be shipped on ice and stored under cold refrigeration conditions with constant rocking in order to maintain intact, viable cells and to preserve cellular and acellular polypeptides, nucleic acid molecules and other analytes from chemical and enzymatic degradation. This requirement for cold storage conditions can limit the availability of whole blood supplies in areas lacking required cold storage facilities, e.g., due to the lack of sufficient equipment or necessary constant electrical power to maintain adequate cold storage temperatures.

Compositions and methods for stabilizing, shipping and storing whole blood and blood components at ambient temperatures have been investigated. While currently used compositions are capable of preventing degradation of polypeptides and nucleic acid molecules, a major limitation is that the stabilized polypeptides and nucleic acid molecules are maintained in a denatured conformation, e.g., single-stranded DNA and unfolded proteins lacking proper secondary and/or tertiary structure, due to the reactive components used in the storage formulations and compositions. A further limitation is that these formulations can result in the lysis of blood cells thereby releasing the cellular contents into the whole blood sample that can make subsequent quantitation and diagnostic analyses of freely circulating polypeptides and nucleic acid molecules complicated due to contaminating genomic DNA and intracellular proteins.

Non-invasive diagnostic tests have been recently developed to determine abnormalities of fetuses using the circulating cell-free DNA in maternal blood (e.g., MATERNIT21® PLUS non-invasive fetal abnormality test, Sequenom, Inc). The impact of this tool has spurned further research in using the cell-free DNA and cell-free amino acid profiles to predict disease states and the relative progression of certain diseases. The use of the collected blood specimens within a short pre-described time is paramount as changes to the profile occur in a relatively short time at ambient temperatures, and exposure to 4°C can cause spurious changes in diagnostic markers on cell surfaces and increase the amounts released into the plasma or serum fractions. While collection tubes for stabilizing cell-free nucleic acids for up to 7 days at room temperatures exist (Streck Labs), the main constituents of the formulations provided in the tubes are formaldehyde releasing agents (such as imidazolinylurea or diazolidinyl urea) that react to fix the cells and make them less permeable to agents entering or exiting the cells.

Thus, there is a need to develop new formulations, compositions and methods for the stabilization of nucleic acid molecules and polypeptides, individually or together, in which the substantially stable nucleic acid molecules and polypeptides are maintained in the blood sample in their native, non-denatured conformation at ambient temperatures. The formulations, compositions and methods of the present disclosure advantageously overcome the aforementioned limitations by maintaining the integrity of blood cells to prevent contamination of circulating nucleic acid molecules and polypeptides by lysed cellular components while also preserving functionally-active polypeptides and nucleic acid molecules in their native conformation at ambient temperatures. These stabilized nucleic acid molecules and polypeptides may be shipped and stored without the need for refrigeration or freezing, and are stable at ambient temperatures for extended periods of time, e.g., days, weeks, months or even years, facilitating the quantitation, analysis and/or use of various nucleic acid molecules and polypeptides for diagnostic and potential therapeutic applications.

### BRIEF SUMMARY

The invention is set out in the appended claims. The embodiments of the description which do not fall within the scope of said claims are provided for illustrative purposes only and do not form part of the present invention. The present disclosure provides formulations for substantially stable storage of one or more polypeptide in a native, non-denatured conformation in a blood sample at ambient temperatures, wherein the one or more polypeptide is stabilized in a native, non-denatured conformation substantially free of contaminating intracellular polypeptides after storage at ambient temperature for a period of at least 3 days. At least 80% of the polypeptides may be stabilized in a native, non-denatured conformation after storage at ambient temperature for a period of at least three days. The one or more polypeptide may remain in a native, non-denatured conformation after storage at ambient temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days. The formulation may comprise: a pH buffer; a non-reducing sugar; a trisaccharide; and one or more of a water-soluble polymer; cationic compound; zwitterionic compound; a phosphatase inhibitor, or a combination thereof. The non-reducing sugar can be sucrose. The trisaccharide may be selected from the group consisting of maltotriose, isomaltotriose, raffinose, melezitose, nigerotriose, and combinations thereof. The trisaccharide can be melezitose. The water-soluble polymer can be polyvinyl alcohol. The phosphatase inhibitor can be 2-glycerol phosphate. The zwitterionic compound can be a compound of formula (I): 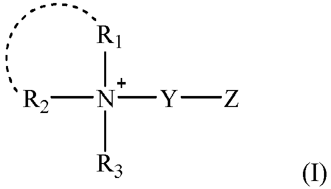 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), wherein A is an unsubstituted or substituted alkyl, aryl, arylalkyl, or any side chain typically found in one of the 20 naturally occurring amino acids; and Z is CO₂-, SO₃- or OPO₃-. The cationic compound may be selected from the group consisting of:
(a) a compound of formula (II): 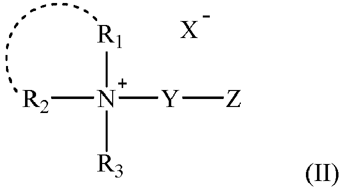 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; Z is CO₂A; and X is a pharmaceutically acceptable anion;
(b) a compound of formula (III): 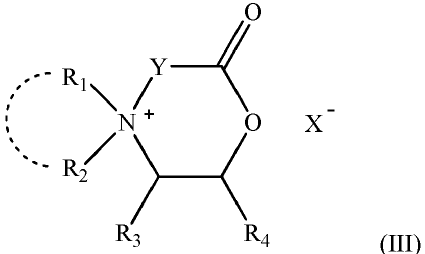 wherein R1, R2, R3, and R4 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; and X is a pharmaceutically acceptable anion; and
(c) a compound of formula (IV): 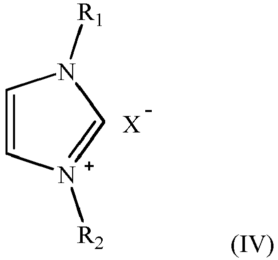 wherein R1 and R2 are independently selected from unsubstituted or substituted alkyl, unsubstituted or substituted aryl, unsubstituted or substituted arylalkyl; and X is a pharmaceutically acceptable anion. R1 and R2 of a compound of formula (I), formula (II), or formula (III) may form a morpholino ring, pyrrolidinium ring, a piperidinium ring, or an oxazinium ring. The zwitterionic compound may be selected from the zwitterionic compounds set forth in Table 1. The cationic compound may be selected from the cationic compounds set forth in Table 1. The zwitterionic compound may be N,N-dimethyl-N-(2-hydroxyethyl)-3-ammonium-proprionate or N-ethyl-piperidinium-4-butylsulfonate. The one or more polypeptide may be selected from the group consisting of an antibody, an enzyme, a plasma protein, a serum protein, and combinations thereof. The composition may comprise: a pH buffer; a non-ionic starch; and one or more of a polyol; a phosphatase inhibitor; an amino acid; or a combination thereof. The polyol may be selected from the group consisting of glycol, glycerol, erythritol, threitol, arabitol, xylitol, ribitol, adonitol, mannitol, sorbitol, galactitol, fucitol, iditol, inositol, and combinations thereof. The polyol may be glycerol. The phosphatase inhibitor may be 2-glycerol phosphate. The amino acid may be glycine. The amino acid may be sarcosine. The non-reducing sugar may be sucrose and the trisaccharide may be selected from the group consisting of maltotriose, isomaltotriose, raffinose, melezitose, and nigerotriose, more preferably melezitose. The water-soluble polymer may be polyvinyl alcohol, the phosphatase inhibitor may be 2-glycerol phosphate, and the zwitterionic compound may be a quaternary inner salt. The water-soluble polymer may be polyvinyl alcohol, the phosphatase inhibitor may be 2-glycerol phosphate, and the zwitterionic compound may be N,N-dimethyl-N-(2-hydroxyethyl)-3-ammonium-proprionate or N-ethyl-piperidinium-4-butylsulfonate. The polyol may be selected from the group consisting of glycol, glycerol, erythritol, threitol, arabitol, xylitol, ribitol, adonitol, mannitol, sorbitol, galactitol, fucitol, iditol and inositol, the phosphatase inhibitor is 2-glycerol phosphate, and the amino acid may be glycine or sarcosine. The polyol may be glycerol, the phosphatase inhibitor may be 2-glycerol phosphate, and the amino acid may be glycine or sarcosine. The formulation may be selected from those set forth in Table 2. There are provided compositions of a substantially, stably stored one or more purified, non-denatured polypeptide comprising one or more purified, non-denatured polypeptide admixed with any of the foregoing formulations.

Described herein are methods for substantially stable storage of one or more polypeptide in a native, non-denatured conformation in a blood sample at ambient temperatures, comprising: admixing a sample of collected blood from a subject with a formulation for substantially stable storage of one or more polypeptide in a native, non-denatured conformation in a blood sample provided herein, wherein the one or more polypeptide remains in a native, non-denatured state after storage at room temperature for a period of at least three days. The polypeptide may be selected from the group consisting of an antibody, an enzyme, a plasma protein, a serum protein, and a combination thereof. At least 80% of the polypeptides may remain in a native, non-denatured state after storage at room temperature for a period of at least three days. The subject may be an animal. The subject may be a mammal. The subject may be a human.

There are provided formulations for substantially stable storage of one or more nucleic acid molecule in a native, non-denatured state in a blood sample at ambient temperatures, wherein the one or more nucleic acid molecule is stabilized in a native, non-denatured state substantially free of contaminating intracellular nucleic acids after storage at ambient temperature for a period of at least 3 days. At least 80% of the nucleic acid molecules may be stabilized in a native, non-denatured state substantially free of contaminating intracellular nucleic acids after storage at room temperature for a period of at least 3 days. The one or more nucleic acid molecule may remain in a native, non-denatured conformation after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days. The formulation may comprise: a pH buffer; a phosphatase inhibitor; a purine; a zwitterionic compound; a cationic compound; or a combination thereof; and an apoptosis inhibitor or a caspase inhibitor. The phosphatase inhibitor may be 2-glycerol phosphate. The zwitterionic compound may be a quaternary inner salt. The quaternary inner salt may be 2-(benzyl(2-hydroxyethyl)(methyl)ammonio)acetate. The zwitterionic compound may be a compound of formula (I): 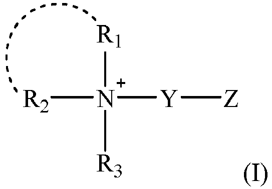 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), wherein A is an unsubstituted or substituted alkyl, aryl, arylalkyl, or any side chain typically found in one of the 20 naturally occurring amino acids; and Z is CO₂-, SO₃- or OPO₃-. The cationic compound may be selected from the group consisting of:
(a) a compound of formula (II): 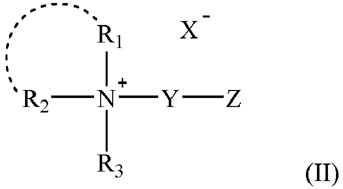 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; Z is CO₂A; and X is a pharmaceutically acceptable anion;
(b) a compound of formula (III): 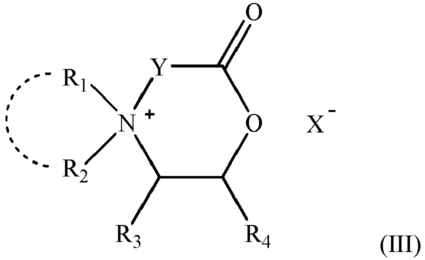 wherein R1, R2, R3, and R4 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; and X is a pharmaceutically acceptable anion; and
(c) a compound of formula (IV): 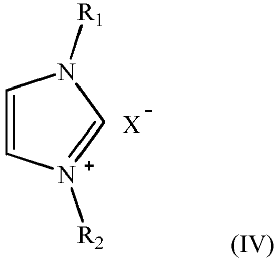 wherein R1 and R2 are independently selected from unsubstituted or substituted alkyl, unsubstituted or substituted aryl, unsubstituted or substituted arylalkyl; and X is a pharmaceutically acceptable anion. R1 and R2 of a compound of formula (I), formula (II), or formula (III) may form a morpholino ring, pyrrolidinium ring, a piperidinium ring, or an oxazinium ring. The zwitterionic compound may be selected from the zwitterionic compounds set forth in Table 1. The cationic compound may be selected from the cationic compounds set forth in Table 1. The formulation may further comprise a nuclease inhibitor. The purine may be adenine. The nuclease inhibitor may be aurin tricarboxylic acid. The composition may further comprise an agent selected from the group consisting of an antibiotic, a purine derivative, and a combination thereof. The antibiotic may be selected from the group consisting of rifampicin, actinomycin D, 5-hydroxy-1,4-napthoquinone, and a combination thereof. The purine derivative may be 5-mercaptopurine. The caspase inhibitor may be Q-VD-OPH. The one or more nucleic acid molecule may be a circulating-free DNA molecule. The phosphatase inhibitor may be 2-glycerol phosphate, the zwitterionic compound may be a quaternary inner salt, more preferably 2-(benzyl(2-hydroxyethyl)(methyl)ammonio)acetate, the apoptosis inhibitor may be Apoptosis Inhibitor (Calbiochem Cat #178488), the caspase inhibitor may be Q-VD-OPH and the pH buffer may be MOPS. The phosphatase inhibitor may be 2-glycerol phosphate, the zwitterionic compound may be 2-(benzyl(2-hydroxyethyl)(methyl)ammonio) acetate, the apoptosis inhibitor may be Apoptosis Inhibitor (Calbiochem Cat #178488), the caspase inhibitor may be Q-VD-OPH and the pH buffer may be MOPS. The formulations may comprise: a pH buffer; a phosphatase inhibitor; a purine; a cationic compound or zwitterionic compound; and a nuclease inhibitor. The phosphatase inhibitor may be 2-glycerol phosphate, the purine may be adenine, the zwitterionic compound may be a quaternary inner salt, the nuclease inhibitor may be aurin tricarboxylic acid and the pH buffer may be MOPS. The phosphatase inhibitor may be 2-glycerol phosphate, the purine may be adenine, the zwitterionic compound may be 2-(benzyl(2-hydroxyethyl)(methyl)ammonio)acetate, the nuclease inhibitor may be aurin tricarboxylic acid and the pH buffer may be MOPS. The formulation may further comprise a polar solvent and a chelating agent. The formulation may further comprise dimethylacetamide and tripotassium EDTA. The formulation may be selected from the formulations set forth in Table 3. There are provided compositions of a substantially, stably stored one or more purified, non-denatured nucleic acid molecule comprising one or more purified, non-denatured nucleic acid molecule admixed with any of the foregoing formulations.

Described herein are methods for substantially stable storage of one or more nucleic acid molecule in a native, non-denatured state in a blood sample at ambient temperatures, comprising: admixing a sample of collected blood from a subject with a formulation for substantially stable storage of one or more nucleic acid molecule in a native, non-denatured state in a blood sample at ambient temperatures provided herein, wherein the one or more nucleic acid molecule remains in a native, non-denatured state substantially free of contaminating intracellular nucleic acids after storage at room temperature for a period of at least three days. The nucleic acid molecule may encode a polypeptide selected from the group consisting of an antibody, an enzyme, and a serum protein an antibody, an enzyme, a plasma protein, and a serum protein. At least 80% of the nucleic acid molecules may remain in a native, non-denatured state substantially free of contaminating intracellular nucleic acids after storage at room temperature for a period of at least three days. The nucleic acid molecule may be a circulating-free DNA molecule. The subject may be an animal. The subject may be a mammal. The subject may be a human.

Described herein are formulations for substantially stable storage of one or more exosome in a native state in a blood sample at ambient temperatures, comprising: a pH buffer; a phosphatase inhibitor; a purine; a zwitterionic compound; a cationic compound; or a combination thereof; and an apoptosis inhibitor or a caspase inhibitor, wherein the one or more exosome is stabilized after storage at room temperature for a period of at least three days. At least 80% of the exosomes may be stabilized after storage at room temperature for a period of at least three days. The one or more exosome may be stabilized after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days. Tthe phosphatase inhibitor may be 2-glycerol phosphate. The zwitterionic compound may be a quaternary inner salt. The quaternary inner salt may be 2-(benzyl(2-hydroxyethyl)(methyl)ammonio)acetate. The zwitterionic compound may be a compound of formula (I): 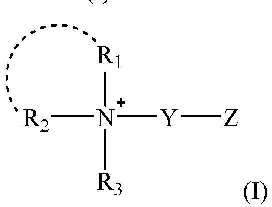 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), wherein A is an unsubstituted or substituted alkyl, aryl, arylalkyl, or any side chain typically found in one of the 20 naturally occurring amino acids; and Z is CO₂-, SO₃- or OPO₃-. The cationic compound may be selected from the group consisting of:
(a) a compound of formula (II): 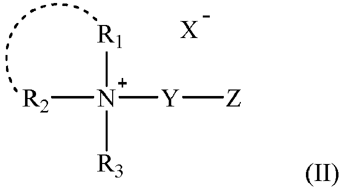 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; Z is CO₂A; and X is a pharmaceutically acceptable anion;
(b) a compound of formula (III): 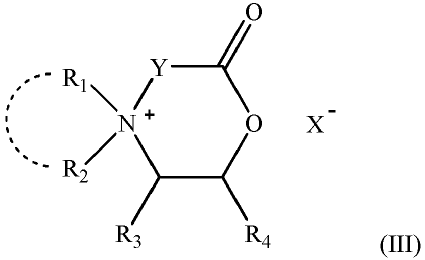 wherein R1, R2, R3, and R4 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; and X is a pharmaceutically acceptable anion; and
(c) a compound of formula (IV): 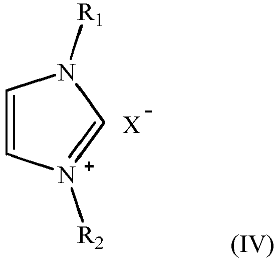 wherein R1 and R2 are independently selected from unsubstituted or substituted alkyl, unsubstituted or substituted aryl, unsubstituted or substituted arylalkyl; and X is a pharmaceutically acceptable anion. R1 and R2 of a compound of formula (I), formula (II), or formula (III) may form a morpholino ring, pyrrolidinium ring, a piperidinium ring, or an oxazinium ring. The zwitterionic compound may be selected from the zwitterionic compounds set forth in Table 1. The cationic compound may be selected from the cationic compounds set forth in Table 1. The formulation may further comprise a nuclease inhibitor. The purine may be adenine. The nuclease inhibitor may be aurin tricarboxylic acid. The composition may further comprise an agent selected from the group consisting of an antibiotic, a purine derivative and a combination thereof. The antibiotic may be selected from the group consisting of rifampicin, actinomycin D, 5-hydroxy-1,4-napthoquinone, and a combination thereof. The purine derivative may be 5-mercaptopurine. The phosphatase inhibitor may be 2-glycerol phosphate, the zwitterionic compound may be a quaternary inner salt, more preferably 2-(benzyl(2-hydroxyethyl)(methyl)ammonio)acetate, the apoptosis inhibitor may be Apoptosis Inhibitor (Calbiochem Cat #178488), the caspase inhibitor may be Q-VD-OPH and the pH buffer may be MOPS. The phosphatase inhibitor may be 2-glycerol phosphate, apoptosis zwitterionic compound may be 2-(benzyl(2-hydroxyethyl)(methyl)ammonio)acetate, the apoptosis inhibitor may be Apoptosis Inhibitor (Calbiochem Cat #178488), the caspase inhibitor may be Q-VD-OPH and the pH buffer may be MOPS. The formulation may comprise a pH buffer, a phosphatase inhibitor, a purine, a cationic compound or zwitterionic compound; and a nuclease inhibitor. The phosphatase inhibitor may be 2-glycerol phosphate, the purine may be adenine, the zwitterionic compound may be a quaternary inner salt, the nuclease inhibitor may be aurin tricarboxylic acid and the pH buffer may be MOPS. The phosphatase inhibitor may be 2-glycerol phosphate, the purine may be adenine, the zwitterionic compound may be 2-(benzyl(2-hydroxyethyl)(methyl)ammonio)acetate, the nuclease inhibitor may be aurin tricarboxylic acid and the pH buffer may be MOPS. The formulation may further comprise a polar solvent and a chelating agent. The formulation may further comprise dimethylacetamide and tripotassium EDTA. The formulation may be selected from the formulations set forth in Table 3. There are provided compositions of a substantially, stably stored one or more purified exosome comprising one or more purified exosome admixed with any of the foregoing formulations.

Described herein are methods for substantially stable storage of one or more exosomes in a native state in a blood sample at ambient temperatures, comprising: admixing a sample of collected blood from a subject with a formulation for substantially stable storage of one or more exosome in a native state in a blood sample at ambient temperatures provided herein, wherein the one or more exosome remains in a native state after storage at room temperature for a period of at least three days. The subject may be an animal. The subject may be a mammal. The subject may be a human.

Described herein are articles of manufacture, comprising any one of the formulations described herein, including those set forth in Tables 2 and 3, contained within a blood collection tube. The blood collection tube may be an evacuated blood collection tube.

Described herein are kits which may comprise any one of the articles of manufacture and a package insert.

### DETAILED DESCRIPTION

The invention is set out in the appended claims. The embodiments of the description which do not fall within the scope of said claims are provided for illustrative purposes only and do not form part of the present invention. The present description relates to formulations, compositions, articles of manufacture, kits and methods for substantially stable storage of one or more polypeptide or nucleic acid molecule in a native, non-denatured conformation in a blood sample at ambient temperatures. The description also relates to formulations, compositions, articles of manufacture, kits and methods for substantially stable storage of one or more exosome in a blood sample at ambient temperatures. The formulations may provide for the substantially stable storage of polypeptides and/or nucleic acid molecules, wherein at least 60%, 70%, 80%, 90%, 95%, or even 99% of the polypeptides and/or nucleic acid molecules remain in a native, non-denatured state after storage at room temperature substantially free of contaminating intracellular components. The formulations may provide for the substantially stable storage of exosomes, wherein at least 60%, 70%, 80%, 90%, 95%, or even 99% of the exosomes are stable after storage at room temperature.

The formulations may provide for substantially stable storage of polypeptides that retain their native conformation and are functionally active. The compositions described herein may also provide substantially stable storage conditions for non-denatured, full-length nucleic acid molecules, such as circulating-free DNA, free of contaminating intracellular DNA for improved quantitation or diagnostic analysis.

### DEFINITIONS

Unless defined otherwise, all technical and scientific terms used herein have the same meaning as is commonly understood by one of skill in the art to which this invention belongs.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

"About" as used herein when referring to a measurable value such as an amount, a temporal duration, and the like, is meant to encompass variations of ±20% or ±10%, or ±5%, or even ±1% from the specified value, as such variations are appropriate for the disclosed compositions or to perform the disclosed methods.

As described herein, a nucleic acid molecule refers to a polymer of two or more modified and/or unmodified deoxyribonucleotides or ribonucleotides, either in the form of a separate fragment or as a component of a larger construction. Nucleic acid molecule(s), oligonucleotide(s), and polynucleotide(s), include RNA or DNA (either single or double stranded, coding, complementary or antisense), or RNA/DNA hybrid sequences of more than one nucleotide in either single chain or duplex form, DNA analogs such as PNA (peptide nucleic acid), and any chemical modifications thereof. The DNA may be a single- or double-stranded DNA, cDNA, or a DNA amplified by any amplification technique, or any DNA polymer. The nucleic acid molecule may be a circulating-free DNA (cfDNA). As used herein, cfDNA refers to DNA that is circulating in a subject's blood and not contained within a cell. The RNA may be mRNA, rRNA, tRNA, siRNA, total RNA, small nuclear RNA (snRNA), RNAi, micro RNA, genomic RNA, RNA isolated from cells or tissues, a ribozyme, or any RNA polymer. Encompassed are native nucleic acid molecules, such as those that can be isolated from natural sources. The nucleic acid molecules may be forms, fragments and derivatives derived from natural sources, as well as recombinant forms and artificial molecules, as long as at least one property of the native molecules is present. The nucleic acid molecules may be within biological samples and may be those that can be applied to analytical, diagnostic and/or pharmaceutical purposes, such as, but not limited to, nucleic acids and their derivatives (e.g. oligonucleotides, DNA, cDNA, PCR products, genomic DNA, plasmids, chromosomes, artificial chromosomes, gene transfer vectors, RNA, mRNA, tRNA, siRNA, miRNA, hnRNA, ribozymes, genomic RNA, peptide nucleic acid (PNA), and bacterial artificial chromosomes (BACs)). The biological sample may be blood.

The term "purified," as used in "purified polypeptide" or "purified nucleic acid molecule" or "purified exosome" refers to recovery of a nucleic acid molecule, a polypeptide, or exosome, respectively, which is at least 50%, at least 55%, at least 60%, at least 65%, at least 70%, at least 75%, at least 80%, at least 85%, at least 90%, at least 92%, at least 94%, at least 95%, at least 96%, at least 98%, % or at least 99% purified with respect to removal of a contaminant, e.g., cellular components such as protein, lipid or salt. The term "substantially purified" generally refers to separation of a majority of cellular proteins or reaction contaminants from the blood sample, so that compounds capable of interfering with the subsequent use of the isolated biomolecule (such as a nucleic acid molecule) are removed.

As described herein, the term "polypeptide" refers to any polymeric chain of amino acids. The terms "peptide" and "protein" are used interchangeably with the term polypeptide and also refer to a polymeric chain of amino acids. The term "polypeptide" encompasses native or artificial proteins, protein fragments and polypeptide analogs of a protein sequence. A polypeptide may be monomeric or polymeric. The term "polypeptide" encompasses fragments and variants (including fragments of variants) thereof, unless otherwise contradicted by context.

The term "isolated protein" or "isolated polypeptide" is a protein or polypeptide that by virtue of its origin (e.g., a cell or biological sample) or source of derivation is not associated with naturally associated components that accompany it in its native state. Thus, a polypeptide that is chemically synthesized will be "isolated" from its naturally associated components. A protein may also be purified or substantially purified by rendering it substantially free of naturally associated components by isolation at least 50%, at least 55%, at least 60%, at least 65%, at least 70%, at least 75%, at least 80%, at least 85%, at least 90%, at least 92%, at least 94%, at least 95%, at least 96%, at least 98%, % or at least 99% purified with respect to removal of a contaminant, e.g., cellular components such as nucleic acids, lipid or salt using protein purification techniques well-known in the art.

### FORMULATIONS AND COMPOSITIONS FOR STABILIZING POLYPEPTIDES, NUCLEIC ACIDS, OR EXOSOMES IN A NATIVE, NON-DENATURED STATE IN A BLOOD SAMPLE AT AMBIENT TEMPERATURES

Formulations and compositions may be provided for substantially stable storage of one or more polypeptide in a non-denatured conformation in a blood sample at ambient temperature, wherein the one or more polypeptide is stabilized in a native, non-denatured conformation after storage at room temperature for a period of at least three days. The formulations and compositions may be provided for substantially stable of polypeptides in a native, non-denatured conformation in a blood sample at ambient temperatures, wherein at least 80% of the polypeptides are stabilized in a native, non-denatured conformation after storage at room temperature for a period of at least three days. The one or more polypeptide may remain in a native, non-denatured conformation after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days.

The term "ambient temperature" as used herein refers to common indoor room temperatures. Ambient temperature may be 15 to 32°C. Ambient temperature may be 20 to 27°C.

The formulations for substantially stable storage of one or more polypeptide and/or nucleic acid molecule in a native, non-denatured state in a blood sample at ambient temperatures may comprise: a pH buffer; a non-reducing sugar; a trisaccharide; and at least one of a water-soluble polymer, a cationic compound, a zwitterionic compound and a phosphatase inhibitor. Formulations for substantially stable storage of one or more polypeptide and/or nucleic acid molecule in a native, non-denatured state in a blood sample at ambient temperatures, may comprise pH buffer; a non-ionic starch; and one or more of a polyol, a phosphatase inhibitor, and an amino acid, wherein the one or more polypeptide and/or nucleic acid molecule is stabilized in a native, non-denatured state after storage at room temperature for a period of at least three days are provided. The polyol may be selected from the group consisting of glycol, glycerol, erythritol, threitol, arabitol, xylitol, ribitol, adonitol, mannitol, sorbitol, galactitol, fucitol, iditol and inositol, the phosphatase inhibitor may be 2-glycerol phosphate, and the amino acid may be glycine or sarcosine. The polyol may be glycerol, the phosphatase inhibitor may be 2-glycerol phosphate, and the amino acid may be glycine or sarcosine.

Formulations may be provided for substantially stable storage of one or more nucleic acid molecule in a native, non-denatured state in a blood sample at ambient temperatures, wherein the one or more nucleic acid molecule remains in a native, non-denatured state substantially free of contaminating intracellular nucleic acids after storage at room temperature for a period of at least three days. Formulations for substantially stable storage of nucleic acid molecules in a native, non-denatured state in a blood sample at ambient temperatures, wherein at least 80% of the nucleic acid molecules are stabilized in a native, non-denatured state after storage at room temperature for a period of at least three days may be provided. The one or more nucleic acid molecule may remain in a native, non-denatured conformation after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days.

The formulations may comprise: a pH buffer; a phosphatase inhibitor; a purine; a zwitterionic compound, a cationic compound, or a combination thereof; and an apoptosis inhibitor or a caspase inhibitor. The phosphatase inhibitor may be 2-glycerol phosphate, the zwitterionic compound may be a quaternary inner salt, more preferably 2-(benzyl(2-hydroxyethyl)(methyl)ammonio)acetate, the apoptosis inhibitor may be Apoptosis Inhibitor (Calbiochem Cat #178488), the caspase inhibitor may be Q-VD-OPH and the pH buffer may be MOPS.

The formulations for substantially stable storage of one or more nucleic acid in a native, non-denatured state in a blood sample at ambient temperature may comprise: a pH buffer; a phosphatase inhibitor; a purine; a zwitterionic compound, a cationic compound, or a combination thereof; and a nuclease inhibitor. The phosphatase inhibitor may be 2-glycerol phosphate, the purine may be adenine, the zwitterionic compound may be a quaternary inner salt, more preferably 2-(benzyl(2-hydroxyethyl)(methyl)ammonio)acetate, the nuclease inhibitor may be aurin tricarboxylic acid and the pH buffer may be MOPS. The formulations may further comprise one of the following: an antibiotic, a purine derivative, an apoptosis inhibitor, a caspase inhibitor, or a combination thereof. The formulation may comprise a polar solvent and a chelating agent. The formulation may further comprise dimethylacetamide and tripotassium EDTA.

Compositions are provided herein in which the blood sample is admixed with the nucleic acid, polypeptide, or exosome and the stabilization formulation to produce substantially stable one or more non-denatured nucleic acid, polypeptide, or exosome, respectively in a whole blood preparation. A composition comprising purified or substantially purified one or more nucleic acid molecule, polypeptide, or exosome admixed with a corresponding stabilization formulation may be provided.

### FORMULATION REAGENTS

### A. pH Buffers

The herein described formulations and compositions for substantially stable storage of a nucleic acid molecule, polypeptide, or exosome in a blood sample at ambient temperatures may include one or more pH buffers. The buffer may be any of a large number of compounds known in the art for their ability to resist changes in the pH of a solution, such as an aqueous solution, in which the pH buffer is present. Selection of one or more particular pH buffers for inclusion in a stable storage composition may be done based on the present disclosure and according to routine practices in the art, and may be influenced by a variety of factors including the pH that is desirably to be maintained, the nature of the sample to be stabilized, the solvent conditions to be employed, the other components of the formulation to be used, and other criteria. For example, typically a pH buffer is employed at a pH that is within about 0.1, 0.2, 0.3, 0.4, 0.5, 0.6, 0.7, 0.8, 0.9 or 1.0 pH unit of a proton dissociation constant (pKₐ) that is a characteristic of the buffer.

Non-limiting examples of pH buffers include citric acid, tartaric acid, malic acid, sulfosalicylic acid, sulfoisophthalic acid, oxalic acid, borate, CAPS (3-(cyclohexylamino)-1-propanesulfonic acid), CAPSO (3-(cyclohexylamino)-2-hydroxy-1-propanesulfonic acid), EPPS (4-(2-hydroxyethyl)-1-piperazinepropanesulfonic acid), HEPES (4-(2-hydroxyethyl)piperazine-1-ethanesulfonic acid), MES (2-(N-morpholino)ethanesulfonic acid), MOPS (3-(N-morpholino)propanesulfonic acid), MOPSO (3-morpholino-2-hydroxypropanesulfonic acid), PIPES (1,4-piperazinediethanesulfonic acid), TAPS (N-[tris(hydroxymethyl)methyl]-3-aminopropanesulfonic acid), TAPSO (2-hydroxy-3-[tris(hydroxymethyl)methylamino]-1-propanesulfonic acid), TES (N-[tris(hydroxymethyl)methyl]-2-aminoethanesulfonic acid), bicine (N,N-bis(2-hydroxyethyl)glycine), tricine (N-[tris(hydroxymethyl)methyl]glycine), tris (tris(hydroxymethyl)aminomethane) and bis-tris (2-[bis(2-hydroxyethyl)amino]-2-(hydroxymethyl)-1,3-propanediol). Certain examples contemplated herein, including a number of those set forth in Table 2 or Table 3, may feature a formulation having a pH of about 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9 or 9.0.

### B. Cationic Compounds and Zwitterionic Compounds

The zwitterionic compound may be a quaternary inner salt. The quaternary inner salts for substantially stable storage of nucleic acids may be those disclosed in WO 2012/018638.

The zwitterionic compound may be a compound of formula (I): 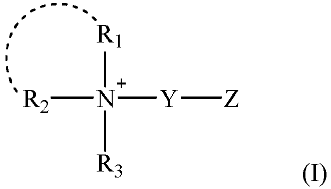 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), wherein A is an unsubstituted or substituted alkyl, aryl, arylalkyl, or any side chain typically found in one of the 20 naturally occurring amino acids; and Z is CO₂-, SO₃- or OPO₃-. R1 and R2 may form a morpholino ring, pyrrolidinium ring, a piperidinium ring, or an oxazinium ring.

The cationic compound may be selected from the group consisting of:
(a) a compound of formula (II): 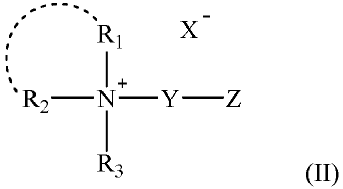 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; Z is CO₂A; and X is a pharmaceutically acceptable anion;
(b) a compound of formula (III): 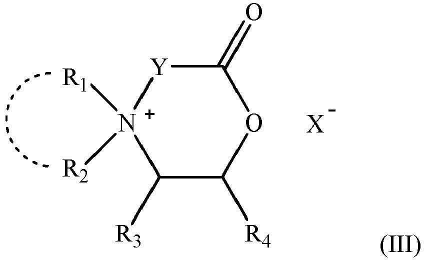 wherein R1, R2, R3, and R4 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; and X is a pharmaceutically acceptable anion; and
(c) a compound of formula (IV): 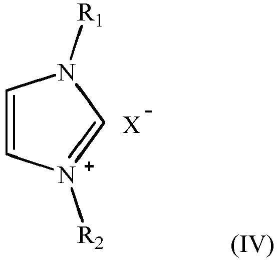 wherein R1 and R2 are independently selected from unsubstituted or substituted alkyl, unsubstituted or substituted aryl, unsubstituted or substituted arylalkyl; and X is a pharmaceutically acceptable anion. R1 and R2 of a compound of formula (II) or formula (III) may form a morpholino ring, pyrrolidinium ring, a piperidinium ring, or an oxaziniumring.

An "alkyl" group refers to an aliphatic hydrocarbon group. The alkyl moiety includes a "saturated alkyl" group, which means that it does not contain any alkene or alkyne moieties. The alkyl moiety also includes an "unsaturated alkyl" moiety, which means that it contains at least one alkene or alkyne moiety. An "alkene" moiety refers to a group that has at least one carbon-carbon double bond, and an "alkyne" moiety refers to a group that has at least one carbon-carbon triple bond. The alkyl moiety, whether saturated or unsaturated, includes branched, straight chain, or cyclic moieties. Depending on the structure, an alkyl group includes a monoradical or a diradical (i.e., an alkylene group), and if a "lower alkyl" having 1 to 6 carbon atoms. As used herein, C1-Cx includes C1-C2, C1-C3 ... C1-Cx. The "alkyl" moiety optionally has 1 to 10 carbon atoms (whenever it appears herein, a numerical range such as "1 to 10" refers to each integer in the given range; e.g., "1 to 10 carbon atoms" means that the alkyl group is selected from a moiety having 1 carbon atom, 2 carbon atoms, 3 carbon atoms, etc., up to and including 10 carbon atoms, although the present definition also covers the occurrence of the term "alkyl" where no numerical range is designated). The alkyl group of the compounds described herein may be designated as "C1-C4 alkyl" or similar designations. By way of example only, "C1-C4 alkyl" indicates that there are one to four carbon atoms in the alkyl chain, i.e., the alkyl chain is selected from among methyl, ethyl, propyl, iso-propyl, n-butyl, iso-butyl, sec-butyl, and t-butyl. Thus C1-C4 alkyl includes C1-C2 alkyl and C1-C3 alkyl. Alkyl groups are optionally substituted or unsubstituted. Typical alkyl groups include, but are in no way limited to, methyl, ethyl, propyl, isopropyl, butyl, isobutyl, tertiary butyl, pentyl, hexyl, ethenyl, propenyl, butenyl, cyclopropyl, cyclobutyl, cyclopentyl, cyclohexyl, and the like.

As used herein, the term "ring" refers to any covalently closed structure. Rings include, for example, carbocycles (e.g., aryls and cycloalkyls), heterocycles (e.g., heteroaryls and non-aromatic heterocycles), aromatics (e.g. aryls and heteroaryls), and non-aromatics (e.g., cycloalkyls and non-aromatic heterocycles). Rings can be optionally substituted. Rings can be monocyclic or polycyclic.

The term "aryl" used alone or as part of a larger moiety as in "arylalkyl", "arylalkoxy", or "aryloxyalkyl", refers to a monocyclic, bicyclic or tricyclic, carbon ring system, that includes fused rings, wherein at least one ring in the system is aromatic. The term "aryl" may be used interchangeably with the term "aryl ring". In one embodiment, aryl includes groups having 6-12 carbon atoms. In another embodiment, aryl includes groups having 6-10 carbon atoms. Examples of aryl groups include phenyl, naphthyl, anthracyl, phenanthrenyl, naphthacenyl, 1,2,3,4-tetrahydronaphthalenyl, 1H-indenyl, 2,3-dihydro-1H-indenyl, and the like. A particular aryl is phenyl. In another embodiment aryl includes indanyl, naphthyl, and tetrahydronaphthyl, and the like, where the radical or point of attachment is on an aromatic ring.

The term "optionally substituted" or "substituted" means that the referenced group may be substituted with one or more additional group(s) individually and independently selected from alkyl, cycloalkyl, aryl, heteroaryl, heteroalicyclic, hydroxy, alkoxy, aryloxy, alkylthio, arylthio, alkylsulfoxide, arylsulfoxide, alkylsulfone, arylsulfone, cyano, halo, acyl, nitro, haloalkyl, fluoroalkyl, amino, including mono and di substituted amino groups, and the protected derivatives thereof. By way of example an optional substituents may be LsRs, wherein each Ls is independently selected from a bond, -O-, -C(=O)-, -S-, -S(=O)-, -S(=O)₂-, -NH-, -NHC(O)-, -C(O)NH-, S(=O)₂NH-, -NHS(=O)₂, -OC(O)NH-, -NHC(O)O-, -(substituted or unsubstituted C1-C6 alkyl), or -(substituted or unsubstituted C2-C6 alkenyl); and each Rs is independently selected from H, substituted or unsubstituted C1-C4 alkyl, substituted or unsubstituted C3-C6 cycloalkyl, heteroaryl, or heteroalkyl.

In certain formulations described herein, including those set forth in Table 2 or Table 3, the cationic compound or zwitterionic compound may be selected from one of the exemplary compounds of Table 1.

**TABLE 1. Exemplary Cationic and Zwitterionic Compounds**

| | | |
|---|---|---|
| D201 | 4-(2-ethoxy-2-oxoethyl)-4-ethylmorpholin-4-ium bromide | Cationic Compound |
| D202 | N-(2-ethoxy-2-oxoethyl)-3-hydroxy-N,N-bis(2-hydroxyethyl)propan-1-aminium bromide | Cationic Compound |
| D203 | 2-ethoxy-N,N,N-triethyl-2-oxoethanaminium bromide | Cationic Compound |
| D204 | 2-((3-hydroxypropyl)dimethylammonio)acetate | Zwitterion |
| D205 | 2-((2-hydroxypropyl)dimethylammonio)acetate | Zwitterion |
| D206 | 2-(2-(hydroxymethyl)-1-methylpiperidinium-1-yl)acetate | Zwitterion |
| D207 | 2-((2-hydroxyethyl)dimethylammonio)acetate | Zwitterion |
| D208 | 2-((2,3-dihydroxypropyl) dimethylammonio)acetate | Zwitterion |
| D209 | 1 -(2-ethoxy-2-oxoethyl)-4-hy droxy-1 -methylpiperidinium bromide | Cationic Compound |
| D210 | 2-(4-hydroxy-1-methylpiperidinium-1-yl)acetate | Zwitterion |
| D211 | 2-ethoxy-N-(2-(2-hydroxyethoxy)ethyl)-N,N-dimethyl-2-oxoethanaminium bromide | Cationic Compound |
| D212 | 2-((2-(2-hydroxyethoxy)ethyl)dimethylammonio)acetate | Zwitterion |
| D213 | 4-(2-hydroxyethyl)-4-methyl-2-oxomorpholin-4-ium bromi de | Cationic Compound |
| D214 | 2-(bis(2-hydroxyethyl)-(methyl)ammonio)acetate | Zwitterion |
| D215 | 2-(4-(2-hydroxyethyl)morpholino-4-ium)acetate | Zwitterion |
| D216 | 2-(4-(2-hydroxyethyl)morpholino-4-ium)acetate | Zwitterion |
| D217 | 4-(2-ethoxy-2-oxoethyl)-4-methylmorpholin-4-ium bromi de | Cationic Compound |
| D218 | 1-(2-ethoxy-2-oxoethyl)-1-methylpyrrolidinium bromide | Cationic Compound |
| D219 | 2-(benzyl(2-hydroxy-ethyl)(methyl)ammonio)acetate | Zwitterion |
| D220 | 3-(2,3-dihydroxypropyl)-1-methyl-1H-imidazol-3-ium chloride | Cationic Compound |
| D221 | 1,3-dimethyl-1H-imidazol-3-ium methyl sulfate | Cationic Compound |
| D222 | 3-(2-ethoxy-2-oxoethyl)-1-methyl-1 H-imidazol-3 -ium bromide | Cationic Compound |
| D223 | 2-(1-(2-hydroxyethyl) pyrrolidinium-1-yl) acetate | Zwitterion |
| D224 | N-benzyl-2-ethoxy-N,N-dimethyl-2-oxoethanaminium bromide | Cationic Compound |
| D225 | 2-ethoxy-N,N-diethyl-N-methyl-2-oxoethanaminium bromide | Cationic Compound |
| D226 | N-(2-ethoxy-2-oxoethyl)-N,N-dimethylbutan-1-aminium bromide | Cationic Compound |
| D227 | 1-(2-ethoxy-2-oxoethyl)-1-methylpiperidinium bromide | Cationic Compound |
| D228 | N-(2-ethoxy-2-oxoethyl)-N,N-dimethylbenzenaminium bromide | Cationic Compound |
| D229 | 1 -(2-ethoxy-2-oxoethyl)-3-hydroxy-1-methylpiperidinium bromide | Cationic Compound |
| D230 | 3-(2-(2-hydroxyethoxy)ethyl)-1-methyl-1H-imidazol-3-ium chloride | Cationic Compound |
| D231 | 3-(2-(2-(2-hydroxyethoxy)ethoxy)ethyl)-1-methyl-1H-imidazol-3-ium chloride | Cationic Compound |
| D232 | 1-methyl-3-tetradecyl-1H-imidazol-3-ium bromide | Cationic Compound |
| D233 | N-(2-ethoxy-2-oxoethyl)-N,N-dimethylcyclohexanaminium bromide | Cationic Compound |
| D234 | 3-((2-hydroxy-ethyl)dimethyl-ammonio)propanoate | Zwitterion |

In certain examples for substantially stable storage of one or more polypeptide, including those set forth in Table 2, the zwitterionic compound is a quaternary inner salt. The quaternary inner salt may be N,N-dimethyl-N-(2-hydroxyethyl)-3-ammonium-proprionate or N-ethyl-piperidinium-4-butylsulfonate, at concentrations of about 1.0 - 100 mg/mL, or 1.0 - 50 mg/mL, or 10.0 - 50 mg/mL. In certain examples for substantially stable storage of one or more nucleic acid molecule, including those set forth in Table 3, the quaternary inner salt is 2-(benzyl(2-hydroxyethyl)(methyl)ammonio)acetate.

### C. Non-reducing sugars

Also described herein are formulations that include at least one non-reducing sugar in the composition for substantially stable storage of one or more polypeptide in a native conformation in a blood sample at ambient temperatures. Non-reducing sugars are carbohydrate molecules that lack a functional aldehyde group. Exemplary non-reducing sugars include sucrose and trehalose. The non-reducing sugar may be trehalose and may be present at a concentration of about 1.0 - 50 mM, or about 10.0 - 30 mM, or about 25 mM. In some examples for substantially stable storage of one or more polypeptide in a native conformation, the non-reducing sugar is sucrose present at a concentration of about 1.0 - 50 mM, or about 1.0 - 30 mM, or about 10 mM.

### D. Trisaccharides

As described herein, the formulations may include at least one trisaccharide in the formulation or composition for substantially stable storage of one or more polypeptide in a native conformation in a whole blood sample at ambient temperatures. Trisaccharides are oligosaccharides composed of three monosaccharide monomers with glycosidic bonds connecting them. The glycosidic bond can be formed between any hydroxyl group on the component monosaccharides and different bond combinations (regiochemistry) and stereochemistry (alpha- or beta-) result in trisaccharides that are diastereoisomers with different chemical and physical properties. Selection of one or more particular trisaccharide for inclusion in a stable storage composition may be done based on the present disclosure and according to routine practices in the art, and may be influenced by a variety of factors including other formulation components. Exemplary trisaccharides include, but are not limited to, maltotriose, isomaltotriose, raffinose, melezitose, and nigerotriose. In certain examples for stabilizing polypeptides, including formulations set forth in Table 2, the trisaccharide is melezitose. The melezitose may be present at a concentration of about 1% - 20%, or about 5.0 - 15 %.

### E. Polyols

Also as described herein, certainexamples include at least one polyol in the composition for substantially stable storage of one or more polypeptide in a native conformation in a whole blood sample at ambient temperatures. Polyols are polyhydric alcohols containing two or more hydroxyl groups and have the general formula H(CHOH)ₙH, wherein n is an integer selected from 2 to 7, inclusive. Polyols differ in chain length with most polyols having five- or six-carbon chains being derived from pentoses (five-carbon sugars) and hexoses (six-carbon sugars); however shorter and longer carbon chain polyols also exist. Exemplary polyols include, but are not limited to, glycol, glycerol, erythritol, threitol, arabitol, xylitol, ribitol, adonitol, mannitol, sorbitol, galactitol, fucitol, iditol and inositol. Selection of one or more particular polyols for inclusion in a substantially stable storage composition may be done based on the present disclosure and according to routine practices in the art, and may be influenced by a variety of factors including other formulation components. The polyol present in the formulation, including those in Table 2, may be a pentose polyol, and may be present at a concentration between 20 - 100 mM, or about 25 - 75 mM. The polyol present in the formulation, including those in Table 2, may be adonitol, and may be present at a concentration between 20 - 100 mM, or about 25 - 75 mM

### F. Water-soluble Polymers

As described herein, certain examples include at least one water-soluble polymer in the formulations and compositions for substantially stable storage of one or more polypeptide in a native conformation in a whole blood sample at ambient temperatures. Such water-soluble polymers include polyvinyl alcohol. It will be appreciated that from the present disclosure the skilled person may select other water-soluble polymers for use in a stable storage formulations and compositions, as may vary based on the other components of the composition that are employed, the particular biological sample being stored, whether nucleic acid molecules or polypeptide molecules or both are sought to be recovered, and other factors. Certain examples, including but not limited to those presented in Table 2, contemplate inclusion of a water-soluble polymer at a concentration (on a volumetric basis, i.e., vol/vol) of about 0.1 to 10% (vol/vol), or about 0.1 to 5% (vol/vol), or 1.0% (vol/vol). The water-soluble polymer may be polyvinyl alcohol with a molecular weight range of about 30-70,000 daltons and about 87-90% hydrolyzed.

### G. Phosphatase Inhibitors

The herein described formulations for substantial stable storage of one or more nucleic acid molecule or polypeptide in a non-denatured state in a blood sample at ambient temperatures may contain a phosphatase inhibitor. The phosphatase inhibitor may be an inhibitor of the serine-threonine class of phosphatases. The phosphatase inhibitor may be 2-glycerol phosphate. For stabilizing polypeptides, the concentration may be about 1.0 - 100 mM, or about 25 - 75 mM, or about 50 mM; and for nucleic acid molecules it may be about 1.0 - 200 mM, or about 25 - 150 mM, or about 125 mM; and for exosomes it may be about 1.0 - 200 mM, or about 25 - 150 mM, or about 125 mM.

### H. Nonionic Starches

The formulations for substantially stable storage of one or more polypeptide in a non-denatured conformation in a blood sample at ambient temperatures may contain a nonionic starch. The nonionic starch may be hydroxyethyl starch (HES). Hydroxyethyl starch is one of the most frequently used volume expanders under the trade names HESPAN by B. Braun Medical Inc. HES may be present at a concentration of about 1.0 - 10%, or about 1.0 - 5.0%, or about 1.0 - 2.0%.

### I. Chelating Agents

Chelating agents or chelators are not included in the presently described formulations and compositions for substantially stable storage of one or more polypeptide or nucleic acid molecule in a non-denatured state in a blood sample, and are known to those familiar with the art for their ability to complex with and hinder the reactivity of metal cations. Exemplary chelating agents include diethylenetriaminepentaacetic acid (DTPA), ethylenediaminetetraacetic acid (EDTA), ethylene glycol tetraacetic acid (EGTA), trans-1,2-diaminocyclohexane-N,N,N',N'-tetraacetic acid (CDTA), 1,2-bis(2-aminophenoxy)ethane-N,N,N',N'-tetraacetic acid (BAPTA), 1,4,7,10-tetraazacyclododecane-1,4,7,10-tetraacetic acid (DOTA), N-(2-hydroxyethyl)ethylenediamine-N,N',N'-triacetic acid, sodium gluconate, and nitrilotriacetic acid (NTA). Exemplary chelating agent concentrations are disodium or tripotassium EDTA present at a concentration of about 1.0 - 100 mM. or about 10 - 90 mM, or about 70 mM.

### J. Nuclease inhibitors

The formulations for substantially stable storage of one or more non-denatured nucleic acid molecule in a blood sample at ambient temperatures may contain a nuclease inhibitor. Nuclease inhibitors are well-known to those skilled in the art. Any such nuclease inhibitor may be used in the formulations, including those in Table 3, compositions and methods of the present disclosure. The nuclease inhibitor may be aurin tricarboxylic acid.

### K. Purine and Purine Derivatives

A purine or purine derivative may be included in the presently described composition for substantially stable storage of nucleic acid molecules in a blood sample. The purine may be adenine, guanine, or a combination thereof. The purine may be a purine derivative. The purine derivative may be 2-mercaptopurine.

### L. Antibiotics

The formulations for substantially stable storage of one or more non-denatured nucleic acid molecule in a blood sample at ambient temperatures may contain an antibiotic. Antibiotics are well-known to those skilled in the art. Any such antibiotic may be used in the formulations, compositions and methods of the present disclosure. The antibiotic may be rifampicin, actinomycin D, 5-hydroxy-1,4-napthoquinone, or a combination thereof.

### M. Polar Solvents

The formulations for substantially stable storage of one or more non-denatured nucleic acid molecule in a blood sample at ambient temperatures may contain a polar solvent. The polar solvent may be dimethylacetamide. The polar solvent may be present at a concentration of 25%. The polar solvent may be dimethylacetamide, and may be present at a concentration of 25%.

### FORMULATIONS TO STABILIZE NON-DENATURED, NATIVE POLYPEPTIDES, NUCLEIC ACID MOLECULES, OR EXOSOMES IN A BLOOD SAMPLE AT AMBIENT TEMPERATURES

Described herein are formulations for substantially stable storage of one or more non-denatured polypeptide in a blood sample at ambient temperature which may comprise: a pH buffer; a non-reducing sugar; a trisaccharide; and at least one of a water-soluble polymer, zwitterionic compound, a cationic compound or a phosphatase inhibitor, wherein the one or more polypeptide and/or nucleic acid molecule remains in a native, non-denatured state after storage at room temperature for a period of at least three days. The one or more polypeptide or nucleic acid molecule may remain in a native, non-denatured conformation substantially free of contaminating intracellular polypeptides after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days.

The disaccharide may be sucrose and the trisaccharide may be selected from the group consisting of maltotriose, isomaltotriose, raffinose, melezitose, and nigerotriose, more preferably melezitose. The water-soluble polymer may be polyvinyl alcohol, the phosphatase inhibitor may be 2-glycerol phosphate, and the zwitterionic compound may be a quaternary amine, preferably N,N-dimethyl-N-(2-hydroxyethyl)-3-ammonium-proprionate or N-ethyl-piperidinium-4-butylsulfonate.

The formulations for substantially stable storage of non-denatured polypeptides in a blood sample at ambient temperatures may be selected from Table 2.

**TABLE 2. Exemplary Formulations for Stabilizing Non-denatured Polypeptides in a Blood Sample at Ambient Temperatures**

| Formulation | Composition |
|---|---|
| A | 10% Sucrose, 6% melezitose 1% polyvinyl alcohol (30-70K mol wt and 87-90% hydrolyzed), |
| | 50 mM Tris pH 8, 1 mM ZnSO₄ , Complete Protease Inhibitor (Roche) |
| B | 10% Sucrose, 6% melezitose 1% polyvinyl alcohol (30-70K mol wt and 87-90% hydrolyzed), |
| | 50 mM Tris pH 8, 25 mg/ mL N,N-dimethyl-N-(2-hydroxyethyl)-3-ammonium-proprionate |
| C | 10% Sucrose, 6% melezitose 1% polyvinyl alcohol (30-70K mol wt and 87-90% hydrolyzed), |
| | 50 mM Tris pH 8, 25 mg/mL N-ethyl-piperidinium-4-butylsulfonate |
| D | 900 mM 2-Glycerol phosphate disodium salt hydrate, 1.8% hydroxyethyl starch, 0.9X PBS |
| E | 2 M Glycine, 0.1% 2-hydroxyethyl starch, 2X PBS, pH 7.4 |
| F | 10% Sucrose, 6% melezitose, 0.5 M 2-glycerol phosphate disodium salt hydrate, 50 mM Tris-HCl, pH 8 |
| G | 0.45 M Sarcosine, 1.35 M glycine, 50 mM Tris-HCl, pH 8 |

Formulations are provided for substantially stable storage of one or more nucleic acid molecule in a native, non-denatured state in a blood sample at ambient temperatures, wherein the one or more nucleic acid molecule is stabilized in a native, non-denatured state substantially free of intracellular nucleic acids after storage at room temperature for a period of at least three days. The formulations may comprise: a pH buffer; a phosphatase inhibitor; a purine; a zwitterionic compound, a cationic compound, or a combination thereof; and an apoptosis inhibitor or a caspase inhibitor. The phosphatase inhibitor may be 2-glycerol phosphate, the zwitterionic compound may be a quaternary inner salt, the apoptosis inhibitor may be Apoptosis Inhibitor (Calbiochem Cat #178488), the caspase inhibitor may be Q-VD-OPH, and the pH buffer may be MOPS. The phosphatase inhibitor may be 2-glycerol phosphate, the zwitterionic compound may be 2-(benzyl(2-hydroxyethyl)(methyl)ammonio) acetate, the apoptosis inhibitor may be Apoptosis Inhibitor (Calbiochem Cat #178488), the caspase inhibitor may be Q-VD-OPH, and the pH buffer may be MOPS. The one or more nucleic acid molecule may remain in a native, non-denatured conformation substantially free of intracellular nucleic acids after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days.

The formulations may comprise: a pH buffer; a phosphatase inhibitor; a purine; a zwitterionic compound, a cationic compound, or a combination thereof; and a nuclease inhibitor. The phosphatase inhibitor may be 2-glycerol phosphate, the purine may be adenine, the zwitterionic compound may be a quaternary inner salt, more preferably 2-(benzyl(2-hydroxyethyl)(methyl)ammonio)acetate, the nuclease inhibitor may be aurin tricarboxylic acid, and the pH buffer may be MOPS. The formulations may further comprise one of the following: an antibiotic, a purine derivative, an apoptosis inhibitor, or a caspase inhibitor. The formulation may further comprise a polar solvent and a chelating agent. The formulation may comprise dimethylacetamide and tripotassium EDTA.

The formulations for substantially stable storage of non-denatured nucleic acid molecules in a blood sample at ambient temperatures may be selected from Table 3.

**TABLE 3. Exemplary Formulations for Stabilizing Non-denatured Nucleic Acid Molecules in a Blood Sample at Ambient Temperatures**

| Formulation | Composition |
|---|---|
| 1 | 500 mM 2-(Benzyl(2-hydroxyethyl)(methyl)ammonio)acetate, 125 mM 2-glycerol phosphate disodium salt, 5 mM adenine, 125 mM MOPS, 4 µM Apoptosis Inhibitor (Calbiochem Cat #178488), pH 6.8 |
| 2 | 500 mM 2-(Benzyl(2-hydroxyethyl)(methyl)ammonio)acetate, 125 mM 2-glycerol phosphate disodium salt, 5 mM adenine, 125 mM MOPS, 4 µM Q-VD-OPH (caspase inhibitor), pH 6.8 |
| 3 | 2.5 M Dihydroxyacetone, 70 mM disodium EDTA, 2 µM Q-VD-OPH (caspase inhibitor) |
| 4 | 25% Dimethylacetamide, 70 mM tripotassium EDTA |
| 5 | 25% Dimethylacetamide, 70 mM tripotassium EDTA, 2 µM Q-VD-OPH (caspase inhibitor) |
| 6 | 500 mM 2-(Benzyl(2-hydroxyethyl)(methyl)ammonio)acetate, 125 mM 2-glycerol phosphate disodium salt, 10 mM adenine, 10 µM aurin tricarboxylic acid, 125 mM MOPS, 10 µM rifampicin, pH 6.8 |
| 7 | 500 mM 2-(Benzyl(2-hydroxyethyl)(methyl)ammonio)acetate, 125 mM 2-glycerol phosphate disodium salt, 10 mM adenine, 10 µM aurin tricarboxylic acid, 125 mM MOPS, 10 µM 6-mercaptopurine monohydrate, pH 6.8 |
| 8 | 500 mM 2-(Benzyl(2-hydroxyethyl)(methyl)ammonio)acetate, 125 mM 2-glycerol phosphate disodium salt, 10 mM adenine, 10 µM aurin tricarboxylic acid, 125 mM MOPS, 10 µM 5-hydroxy-1,4-napthoquinone, pH 6.8 |
| 9 | 500 mM 2-(Benzyl(2-hydroxyethyl)(methyl)ammonio)acetate, 125 mM 2-glycerol phosphate disodium salt, 10 mM adenine, 10 µM aurin tricarboxylic acid, 125 mM MOPS, 10 µM Actinomycin D, pH 6.8 |
| 10 | 500 mM 2-(Benzyl(2-hydroxyethyl)(methyl)ammonio)acetate, 125 mM 2-glycerol phosphate disodium salt, 10 mM adenine, 10 µM aurin tricarboxylic acid, 125 mM MOPS, 10 µM Q-VD-OPH (caspase inhibitor), pH 6.8 |

Formulations are provided for substantially stable storage of one or more exosome in a native state in a blood sample at ambient temperatures for a period of at least three days. The formulations may comprise: a pH buffer; a phosphatase inhibitor; a purine; a zwitterionic compound, a cationic compound, or a combination thereof; and an apoptosis inhibitor or a caspase inhibitor. The phosphatase inhibitor may be 2-glycerol phosphate, the zwitterionic compound may be a quaternary inner salt, the apoptosis inhibitor may be Apoptosis Inhibitor (Calbiochem Cat #178488), the caspase inhibitor may be Q-VD-OPH, and the pH buffer may be MOPS. The phosphatase inhibitor may be 2-glycerol phosphate, the zwitterionic compound may be 2-(benzyl(2-hydroxyethyl)(methyl)ammonio) acetate, the apoptosis inhibitor may be Apoptosis Inhibitor (Calbiochem Cat #178488), the caspase inhibitor may be Q-VD-OPH, and the pH buffer may be MOPS. The exosome may remain in a native state after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days. The formulations may comprise: a pH buffer; a phosphatase inhibitor; a purine; a zwitterionic compound or cationic compound; and a nuclease inhibitor. The phosphatase inhibitor may be 2-glycerol phosphate, the purine may be adenine, the zwitterionic compound may be a quaternary inner salt, more preferably 2-(benzyl(2-hydroxyethyl)(methyl)ammonio)acetate, the nuclease inhibitor may be aurin tricarboxylic acid, and the pH buffer may be MOPS. The formulations may further comprise one of the following: an antibiotic and a purine derivative. The formulation may further comprise a polar solvent and a chelating agent. The formulation may comprise dimethylacetamide and tripotassium EDTA.

The formulation for substantially stable storage exosomes in a blood sample at ambient temperatures may be selected from Table 3.

### METHODS FOR PREPARING FORMULATIONS FOR STABILIZING NON-DENATURED NUCLEIC ACID MOLECULES AND POLYPEPTIDES AT AMBIENT TEMPERATURES

The present formulations may be prepared using commercially available reagents and methods well-known to those skilled in the art. The formulations may be prepared as concentrated stock solutions of the formulation reagents, e.g., 2X, 5X, 10 X, 20X or the like, and admixed with the blood sample at the appropriate concentrations. The blood sample may be admixed with the formulation stock at an equal volume (1:1).

### PURIFIED NUCLEIC ACID MOLECULES AND POLYPEPTIDES

The substantially stable one or more polypeptide and/or nucleic acid molecule in a blood sample at ambient temperatures may be further purified using well-known conventional methods routinely employed by those skilled in the art. Apparatus, kits, and methods for purifying nucleic acid molecules and polypeptides from blood are well-known. For instance, substantially stabilized polypeptides and nucleic acids are purified, for example, by affinity chromatography, gel electrophoresis, or the like. The purified one or more polypeptide or nucleic acid molecule may be subsequently stored in the formulations described herein for extended periods before analysis.

### ARTICLES OF MANUFACTURE

Articles of manufacture are provided in which one of the herein described formulations, including those set forth in Tables 2 or 3, may be contained within a suitable blood collection tube, container or vessel. These articles of manufacture may be used for substantially stable storage of one or more blood component by stabilizing one or more blood component at the time of blood collection. The blood collection tube may be an evacuated blood tube having less than atmospheric pressure to withdraw a predetermined volume of whole blood. These articles of manufacture may be used in the herein described kits and methods.

### KITS

There are provided kits comprising any one of the articles of manufacture comprising the formulations of the present disclosure and a package insert. The components of the kit may be supplied in a container, such as a compartmentalized plastic enclosure. The container may have a hermetically sealable cover so that the contents of the kit can be sterilized and sealed for storage prior to use.

### METHODS FOR SUBSTANTIALLY STABLE STORAGE OF BLOOD COMPONENTS

Methods for substantially stable storage of one or more polypeptide, nucleic acid molecule, or exosome in a blood sample in a native, non-denatured conformation or state may be provided.

The methods may comprise admixing a blood sample with a formulation for substantially stable storage of one or more polypeptide in a blood sample at ambient temperatures, wherein the one or more polypeptide is stabilized in a native, non-denatured state after storage at room temperature for a period of at least three days. At least 80% of the polypeptides may remain in a native, non-denatured state for a period of at least three days. The formulation for substantially stable storage of one or more non-denatured polypeptide in a blood sample at ambient temperatures may comprise: a pH buffer; a non-reducing sugar; a trisaccharide; and at least one or more of a water-soluble polymer, a zwitterionic compound, a cationic compound or a phosphatase inhibitor. The formulation may be one of the formulations set forth in Table 2. In the method, the one or more polypeptide may remain in a native, non-denatured conformation after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days.

The methods may comprise admixing a blood sample with formulations for substantially stable storage of one or more nucleic acid molecule in a blood sample at ambient temperatures, wherein the one or more nucleic acid molecule is stabilized in a native, non-denatured state substantially free of contaminating intracellular nucleic acids after storage at room temperature for a period of at least three days. At least 80% of the nucleic acids may remain in a native, non-denatured state for a period of at least three days. The formulation for substantially stable storage of one or more non-denatured nucleic acid molecule in a blood sample at ambient temperature may comprise: a pH buffer; a phosphatase inhibitor; a purine; a zwitterionic compound, a cationic compound, or a combination thereof; and an apoptosis inhibitor or a caspase inhibitor. The formulation may comprise: a pH buffer; a phosphatase inhibitor; a purine; a zwitterionic compound or cationic compound; and a nuclease inhibitor. The formulation may further comprise: a polar solvent and a chelating agent. The formulation may further comprise dimethylacetamide and tripotassium EDTA. The formulation may be one of the formulations set forth in Table 3. The one or more nucleic acid molecule may remain in a native, non-denatured conformation after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days.

The methods may comprise admixing a blood sample with formulations for substantially stable storage of one or more exosome in a blood sample at ambient temperatures, wherein the one or more exosome is stabilized for a period of at least three days at ambient temperature. At least 80% of the exosomes may be stabilized for a period of at least three days. The formulation for substantially stable storage of one or more exosome in a blood sample at ambient temperature may comprise: a pH buffer; a phosphatase inhibitor; a purine; a zwitterionic compound, a cationic compound, or a combination thereof; and an apoptosis inhibitor or a caspase inhibitor. The formulation may comprise: a pH buffer; a phosphatase inhibitor; a purine; a zwitterionic compound or cationic compound; and a nuclease inhibitor. The formulation may further comprise: a polar solvent and a chelating agent. The formulation may further comprise dimethylacetamide and tripotassium EDTA. The formulation may be one of the formulations set forth in Table 3. The one or more exosome may be stabilized after storage at room temperature for a period of at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days.

Blood collection tubes, bags, containers and vessels are well-known in the art and have been employed by medical practitioners for decades. Blood may be collected using any method or apparatus commonly employed by those skilled in the art such as venipuncture or finger prick. When the blood is collected by venipuncture, the formulations of the invention may be located inside the blood collection tube, e.g., an evacuated tube (VACUTAINER blood collection tube, Becton Dickenson or VACUETTE, Greiner) at the time that the blood sample is obtained from the subject, or the formulations may be added to an already obtained whole blood sample, preferably immediately or shortly after it is withdrawn.

The methods as described herein may use the articles of manufacture and kits disclosed herein.

The following Examples are presented by way of illustration and not limitatior Example 1 does not illustrate the claimed invention.

### EXAMPLE 1

### STABILIZATION OF FUNCTIONALLY ACTIVE POLYPEPTIDES IN A HUMAN BLOOD SAMPLE FOR A PERIOD OF AT LEAST NINE DAYS AT AMBIENT TEMPERATURES

This Example demonstrated formulations, including those set forth for preparing substantially stable storage of native, functionally active polypeptides in a native, non-denatured conformation in a blood sample for a period of at least nine days at ambient temperatures.

The ability of the exemplary formulations A - G set forth in Table 2 to stabilize a plurality of polypeptides, e.g., a survey of 29 cytokines and chemokines, at ambient temperatures was examined using Millipex MAP Human cytokine/chemokine panel HCYTOMAG-60K-PX29 kit from Luminex Corp in accordance with the manufacturer's instructions.

Briefly, human whole blood samples were collected from donors and the blood was fractionated and the plasma layer was analyzed. A 75 or 135 (9:1 samples in Table 2) microliter aliquot of plasma was added to a 1.7 mL microfuge tube to which a solution of 29 different cytokines was added to spike the samples with an internal standard of 400 pg/mL, and the volume adjusted to 150 microliter. Control samples containing the internal standard in the absence (stored at -80°C) or presence of plasma (fresh on assay day) were prepared and processed simultaneously with test samples. In addition, a standard curve was prepared using 16, 80, 400, 2,000, and 10,000 pg/mL, to ensure the linearity of the assay and to assist in proper quantitation of bound cytokine and chemokine concentrations.

Control and test samples were placed at room temperature and 25 microliter aliquots were removed at Day 0, Day 3 and Day 6. Day 0 and Day 3 samples were stored at -80°C until processed on Day 6. To each 25 mL aliquot, 25 microliters each of Assay Buffer was added followed by the addition of 25 microliter amount of a human cytokine/chemokine antibody-immobilized magnetic beads coated with one of 29 antibodies directed against each member of the cytokine/chemokine panel was added. The antibodies recognize only non-denatured cytokine and chemokine polypeptides and denatured polypeptides will not bind to the beads. The mixtures were incubated overnight at 4°C with shaking. The 96-well plate was placed against a magnet to sequester the bound magnetic beads, the supernatant was removed and the beads were washed twice with wash buffer.

To the washed beads, a 25 microliter aliquot of a Detection Antibody solution was added to each sample and incubated for one hour at room temperature. A 25 microliter aliquot of a streptavidin-phycoerythrin solution was added and the samples were stored at room temperature for 30 min. A magnet was placed next to the 96-well plate to sequester the beads, the supernatants were removed, and the samples were washed twice and resuspended in 150 microliters of wash buffer.

The amount of bound cytokine/chemokine from each plasma sample was determined using the Luminex Bio-Tek ELx405 instrument according to the manufacturer's instruction and the amount of each of the 29 members of the non-denatured cytokines and chemokines for each sample was calculated. The results for a subset of cytokines are shown in Table 4.

**Table 4**

| Stabilization of Panel of Cytokines and Chemokines from a Blood Sample at Ambient Temperatures Using the Formulations of Table 2. | | | | | |
|---|---|---|---|---|---|
| Cytokine | Formulation | Day 0 | Day 3 | Day 6 | Day 14 |
| IFN 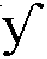 | NP | 100 | 78.10948 | 81.63109 | 19.41903 |
| | F | 100 | 106.5608 | 102.5185 | 85.24264 |
| IL-13 | NP | 100 | 80.85405 | 79.72953 | 45.60758 |
| | A | 100 | 105.8785 | 95.30526 | 70.70471 |
| | D | 100 | 89.69889 | 102.5402 | 77.19855 |
| | E | 100 | 105.8785 | 95.30526 | 70.70471 |
| | G | 100 | 89.69889 | 102.5402 | 77.19855 |
| TNFα | NP | 100 | 67.11964 | 51.49416 | 19.58567 |
| | A | 100 | 104.9812 | 80.6713 | 52.96681 |
| | G | 100 | 82.17989 | 69.07483 | 52.29539 |

| | | | | | |
|---|---|---|---|---|---|
| NP: Non-protected control | | | | | |

### EXAMPLE 2

### STABILIZATION OF CIRCULATING FREE NUCLEIC ACIDS IN HUMAN BLOOD SAMPLE FOR A PERIOD OF AT LEAST EIGHT DAYS AT AMBIENT TEMPERATURES

This Example demonstrated formulations, including those set forth Table 3, for preparing substantially stable storage of nucleic acid molecules in a native, non-denatured state in a blood sample while preventing substantial contamination from intracellular nucleic acids from lysed blood cells for a period of at least eight days at ambient temperatures.

The ability of the exemplary Formulations 1 - 10 set forth in Table 3 to stabilize a plurality circulating-free nucleic acids that are substantially free of contaminating intracellular nucleic acids in a blood sample was examined in two separate experiments. Briefly, 1.0 mL of the formulation was placed into three 5 mL Eppendorf tubes for each formulation, respectively. The non-protected tubes at room temperature and 4°C received no formulation. Fresh human whole blood was collected from a healthy donor. The blood was pooled, mixed and then 4.0 mL aliquoted into each tube. After 10 tubes were loaded with blood the tubes were closed and mixed by inversion 10 times before adding blood to more tubes. The blood was remixed before addition to the next set of tubes. Once all the tubes were loaded and mixed the time zero tubes were spun at 3000 rpm for 10 minutes. Two 1.25 mL aliquots of the plasma were removed without disturbing the leukocyte and red blood cells, transferred to individual 1.5 mL Eppendorf microfuge tubes and spun at 16,000 g for 10 min at 4°C. The NP room temperature time zero blood was prepared by addition of 1 mL of 1X PBS pH 7.4. The tube was mixed by inversion 10 times and then the plasma prepared as described above. The two 1 mL cell-free plasma aliquots for each formulation tested were extracted using the MagNA Pure Compact Nucleic Acid Isolation Kit 1, Large Volume (Roche Cat# 03 730 972 001) on the Roche MagNA Pure Compact Instrument from Roche Molecular Systems. The nucleic acids were eluted in 100 µL of elution buffer. The time zero samples were held at 4°C until the day 4 samples were prepared as described above. The DNA extracted from each sample was analyzed by qPCR on a Bio-Rad CFX96 Real Time System (C1000 Touch Thermal Cycler) using a set of 18s rRNA primers from Qiagen (Cat# PPH05666E-200) and the Bio-Rad iQ SYBR Green Supermix (Cat# 170-8882). 5 µL of each sample was used and the following plot generated. In addition, a 1:10 dilution of the isolated DNA was prepared and 5 µL of the dilution was also amplified by qPCR.

**Table 5**

| Stabilization Circulating-Free Nucleic Acids Substantially Free of Contaminating Intracellular Nucleic Acids in a Blood Sample at Ambient Temperatures Using the Formulations of Table 3. Yield (µg/mL) | | | | | | | |
|---|---|---|---|---|---|---|---|
| Day | 4°C | RT | Form 1 | Form 2 | Form 3 | Form 4 | Form 5 |
| 0 | 0.02 | 0.02 | 0.02 | 0.02 | 0.02 | 0.02 | 0.02 |
| 4 | 0.17 | 0.19 | 0.14 | 0.04 | 0.02 | 0.07 | 0.07 |
| 8 | 0.06 | 0.91 | 0.10 | 0.06 | 0.08 | 0.03 | 0.08 |

**Table 6**

| Stabilization Circulating-Free Nucleic Acids Substantially Free of Contaminating Intracellular Nucleic Acids in a Blood Sample at Ambient Temperatures Using the Formulations of Table 3. Yield (µg/mL) | | | | | | | |
|---|---|---|---|---|---|---|---|
| Day | 4°C | RT | Form 6 | Form 7 | Form 8 | Form 9 | Form 10 |
| 0 | 0.03 | 0.03 | 0.03 | 0.03 | 0.03 | 0.03 | 0.03 |
| 4 | 0.29 | 0.44 | 0.42 | 0.43 | 0.34 | 0.43 | 0.08 |
| 8 | 3.01 | 4.21 | 0.77 | 0.65 | 0.76 | 0.69 | 0.11 |

As shown in Tables 5 and 6, the addition of the exemplary formulations of Table 3 to human blood resulted in recovery of circulating-free nucleic acids that remained substantially free of contaminating intracellular DNA. At Day 0, the amount of circulating-free nucleic acids present in the test samples was essentially identical to controls and to other test samples; however, at Day 8, the blood stored at 4°C or room temperature showed a significant increase the level of circulating-free nucleic acids shown by the increased yield in these samples from contaminating intracellular nucleic acids from lysed blood cells. In contrast, formulations 1-10 maintained the circulating-free nucleic acids substantially free of intracellular nucleic acids showing only modest increases at Day 8 and all of these formulations performed about as well or better than unprotected samples stored for 8 days at 4°C.

Unless the context requires otherwise, throughout the present specification and claims, the word "comprise" and variations thereof, such as, "comprises" and "comprising," which is used interchangeably with "including," "containing," or "characterized by," is inclusive or open-ended language and does not exclude additional, unrecited elements or method steps. The phrase "consisting of" excludes any element, step, or ingredient not specified in the claim. The phrase "consisting essentially of" limits the scope of a claim to the specified materials or steps and those that do not materially affect the basic and novel characteristics of the claimed invention.

## Claims

1. A formulation for substantially stable storage of one or more nucleic acid molecule in a native, non-denatured state in a blood sample at ambient temperatures, wherein the one or more nucleic acid molecule is stabilized in a native, non-denatured state substantially free of contaminating intracellular nucleic acids after storage at room temperature for a period of at least four days, wherein the formulation comprises:
(i) a pH buffer;
(ii) a phosphatase inhibitor;
(iii) a purine;
(iv) a zwitterionic compound, a cationic compound, or a combination thereof; and
(v) a compound selected from:
(v.a) an apoptosis inhibitor or a caspase inhibitor; and/or
(v.b) a nuclease inhibitor;
and wherein said formulation does not comprise a chelating agent, such as EDTA.

2. The formulation of Claim 1, wherein the phosphatase inhibitor is 2-glycerol phosphate.

3. The formulation of Claims 1 or 2,
i) wherein the zwitterionic compound is a quaternary inner salt, 2-(benzyl(2-hydroxyethyl)(methyl)ammonio)acetate, N,N-dimethyl-N-(2-hydroxyethyl)-3-ammonium- proprionate, or N-ethyl-piperidinium-4-butylsulfonate; or a compound of formula (I): 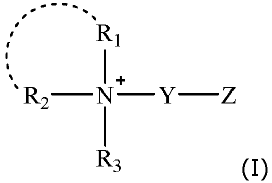 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), wherein A is an unsubstituted or substituted alkyl, aryl, arylalkyl, or any side chain typically found in one of the 20 naturally occurring amino acids; and Z is CO₂-, SO₃- or OPO₃-; or
ii) wherein the cationic compound is selected from the group consisting of:
(a) a compound of formula (II): 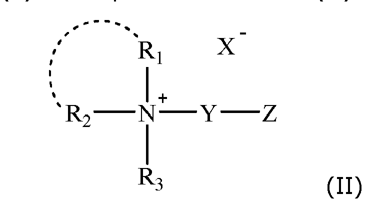 wherein R1, R2, and R3 are independently selected from unsubstituted or substituted alkyl, unsubstituted or substituted aryl, unsubstituted or substituted arylalkyl, or RI and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; Z is CO₂A; and X is a pharmaceutically acceptable anion;
(b) a compound of formula (III): 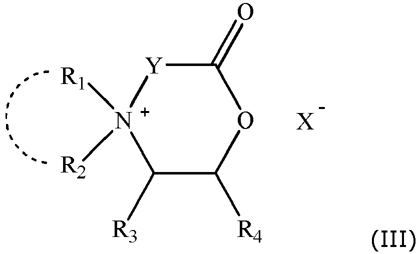 wherein R1, R2, R3, and R4 are independently selected from unsubstituted or substituted alkyl, unsubstituted or substituted aryl, unsubstituted or substituted arylalkyl, or RI and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; and X is a pharmaceutically acceptable anion; and
(c) a compound of formula (IV): 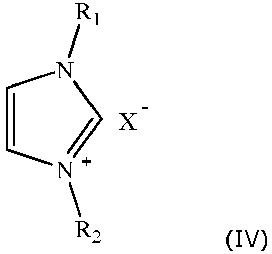 wherein R1 and R2 are independently selected from unsubstituted or substituted alkyl, unsubstituted or substituted aryl, unsubstituted or substituted arylalkyl; and X is a pharmaceutically acceptable anion.

4. The formulation of Claim 3, wherein R1 and R2 of a compound of formula (I), formula (II), or formula (III) form a morpholino ring, pyrrolidinium ring, a piperidinium ring, or an oxazinium ring.

5. The formulation of any one of Claims 1-4, wherein
• the nuclease inhibitor is aurin tricarboxylic acid;
• the purine is adenine; and/or
• the caspase inhibitor is Q-VD-OPH.

6. The formulation of any one of Claims 1-5, further comprising an agent selected from the group consisting of an antibiotic, a purine derivative, and a combination thereof.

7. The formulation of Claim 6, wherein
• the antibiotic is selected from the group consisting of rifampicin, actinomycin D, 5-hydroxy-1,4-napthoquinone, and a combination thereof;
• the purine derivative is 5-mercaptopurine.

8. The formulation of any one of Claims 1-7, wherein the one or more nucleic acid molecule is a circulating-free DNA molecule.

9. A composition of a substantially, stably stored one or more purified, non-denatured nucleic acid molecule comprising one or more purified, non-denatured nucleic acid molecule admixed with a formulation of any one of Claims 1-8.

10. A method for substantially stable storage of one or more nucleic acid molecule in a native, non-denatured state in a blood sample at ambient temperatures, comprising: admixing a sample of collected blood from a subject with a formulation of any one of Claims 1-8, wherein the one or more nucleic acid molecule remains in a native, non-denatured state substantially free of contaminating intracellular nucleic acids after storage at room temperature for a period of at least three days.

11. The method of Claim 10, wherein the nucleic acid molecule encodes a polypeptide selected from the group consisting of an antibody, an enzyme, and a serum protein, an antibody, an enzyme, a plasma protein, and a serum protein.

12. The method of any one of Claims 10-11, wherein the nucleic acid molecule is a circulating- free DNA molecule.

## Patentansprüche

1. Formulierung zur im Wesentlichen stabilen Lagerung eines Nukleinsäuremoleküls oder mehrerer Nukleinsäuremoleküle in einem nativen, nicht denaturierten Zustand in einer Blutprobe bei Umgebungstemperaturen, wobei das eine Nukleinsäuremolekül oder die mehreren Nukleinsäuremoleküle stabilisiert sind in einem nativen, nicht denaturierten Zustand im Wesentlichen frei von kontaminierenden intrazellulären Nukleinsäuren nach der Lagerung bei Raumtemperatur für eine Dauer von mindestens vier Tagen, wobei die Formulierung umfasst:
(i) einen pH-Puffer;
(ii) einen Phosphataseinhibitor;
(iii) ein Purin;
(iv) eine zwitterionische Verbindung, eine kationische Verbindung oder eine Kombination davon; und
(v) eine Verbindung, ausgewählt aus:
(v.a) einem Apoptose-Inhibitor oder einem Caspase-Inhibitor; und/oder
(v.b) einem Nuklease-Inhibitor;
und wobei die Formulierung keinen Chelatbildner, wie beispielsweise EDTA, umfasst.

2. Formulierung nach Anspruch 1, wobei der Phosphataseinhibitor 2-Glycerinphosphat ist.

3. Formulierung nach den Ansprüchen 1 oder 2,
i) wobei die zwitterionische Verbindung folgendes ist: ein inneres Salz, 2-(Benzyl(2-hydroxyethyl)(methyl)ammonio)acetat, N,N-Dimethyl-N-(2-hydroxyethyl)-3-ammonium- proprionat oder N-Ethyl-piperidinium-4-butylsulfonat; oder eine Verbindung der Formel (I): 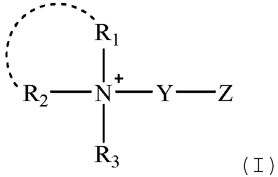 wobei R1, R2 und R3 unabhängig voneinander ausgewählt sind aus nicht substituiertem oder substituiertem Alkyl, nicht substituiertem oder substituiertem Aryl, nicht substituiertem oder substituiertem Arylalkyl oder R1 und R2 optional einen Ring bilden, Y ist CH₂, CH(A), CH(A)-CH(A), CH(A)-CH(A)-CH(A), wobei A ein nicht substituiertes oder substituiertes Alkyl, Aryl, Arylalkyl oder eine beliebige Seitenkette ist, die typischerweise in einer der 20 natürlich vorkommenden Aminosäuren gefunden wird; und Z ist CO₂-, SO₃- oder OPO₃-; oder
ii) wobei die kationische Verbindung ausgewählt ist aus der Gruppe bestehend aus:
**(a)** einer Verbindung der Formel (II): 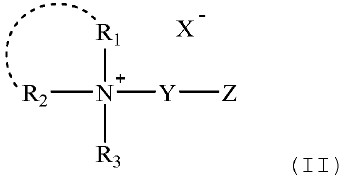 wobei R1, R2 und R3 unabhängig voneinander ausgewählt sind aus nicht substituiertem oder substituiertem Alkyl, nicht substituiertem oder substituiertem Aryl, nicht substituiertem oder substituiertem Arylalkyl, oder R1 und R2 optional einen Ring bilden, Y ist CH₂, CH(A), CH(A)-CH(A), CH(A)-CH(A)-CH(A), wobei A ein nicht substituiertes oder substituiertes Alkyl, Aryl, Arylalkyl oder eine beliebige Seitenkette ist, die typischerweise in einer der 20 natürlich vorkommenden Aminosäuren gefunden wird; Z ist CO₂A; und X ist ein pharmazeutisch akzeptables Anion;
**(b)** einer Verbindung der Formel (III): 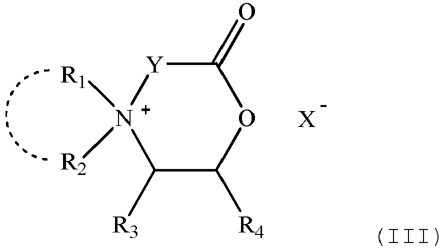 wobei R1, R2, R3 und R4 unabhängig voneinander ausgewählt sind aus nicht substituiertem oder substituiertem Alkyl, nicht substituiertem oder substituiertem Aryl, nicht substituiertem oder substituiertem Arylalkyl oder R1 und R2 optional einen Ring bilden, Y ist CH₂, CH(A), CH(A)-CH(A), CH(A)-CH(A)-CH(A), wobei A ein nicht substituiertes oder substituiertes Alkyl, Aryl, Arylalkyl oder eine beliebige Seitenkette ist, die typischerweise in einer der 20 natürlich vorkommenden Aminosäuren gefunden wird; und X ist ein pharmazeutisch akzeptables Anion; und
**(c)** einer Verbindung der Formel (IV): 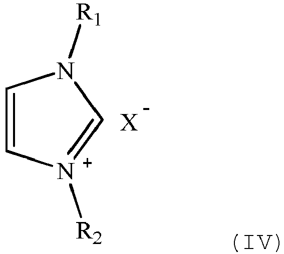 wobei R1 und R2 unabhängig voneinander ausgewählt sind aus nicht substituiertem oder substituiertem Alkyl, nicht substituiertem oder substituiertem Aryl, nicht substituiertem oder substituiertem Arylalkyl; und X ist ein pharmazeutisch verträgliches Anion.

4. Formulierung nach Anspruch 3, wobei R1 und R2 eine Verbindung der Formel (I), Formel (II) oder Formel (III) ein Morpholinoring, Pyrrolidiniumring, ein Piperidiniumring oder ein Oxaziniumring sind.

5. Formulierung nach einem der Ansprüche 1-4, wobei
• der Nuklease-Inhibitor Aurin-Tricarbonsäure ist;
• das Purin Adenin ist; und/oder
• der Caspase-Inhibitor Q-VD-OPH ist.

6. Formulierung nach einem der Ansprüche 1-5, die ferner ein Mittel umfasst, das aus der Gruppe ausgewählt ist, die aus einem Antibiotikum, einem Purinderivat und einer Kombination davon besteht.

7. Formulierung nach Anspruch 6, wobei
• das Antibiotikum ausgewählt ist aus der Gruppe bestehend aus Rifampicin, Actinomycin D, 5-Hydroxy-1,4-napthochinon und einer Kombination davon;
• das Purinderivat 5-Mercaptopurin ist.

8. Formulierung nach einem der Ansprüche 1-7, wobei das eine Nukleinsäuremolekül oder die mehreren Nukleinsäuremoleküle ein zirkulationsfreies DNA-Molekül ist.

9. Zusammensetzung eines im Wesentlichen stabil gelagerten einen oder mehrerer gereinigten, nicht denaturierten Nukleinsäuremoleküle, umfassend ein oder mehrere gereinigte, nicht denaturierte Nukleinsäuremoleküle, die mit einer Formulierung nach einem der Ansprüche 1-8 gemischt sind.

10. Verfahren zur im Wesentlichen stabilen Lagerung eines oder mehrerer Nukleinsäuremoleküle in einem nativen, nicht denaturierten Zustand in einer Blutprobe bei Umgebungstemperaturen, umfassend: Mischen einer von einem Subjekt gesammelten Blutprobe mit einer Formulierung nach einem der Ansprüche 1-8, wobei das eine Nukleinsäuremolekül oder die mehreren Nukleinsäuremoleküle in einem nativen, nicht denaturierten Zustand im Wesentlichen frei von kontaminierenden intrazellulären Nukleinsäuren verbleiben, nach Lagerung bei Raumtemperatur für einen Zeitraum von mindestens drei Tagen.

11. Verfahren nach Anspruch 10, wobei das Nukleinsäuremolekül ein Polypeptid kodiert, das aus der Gruppe ausgewählt ist, die aus einem Antikörper, einem Enzym und einem Serumprotein, einem Antikörper, einem Enzym, einem Plasmaprotein und einem Serumprotein besteht.

12. Verfahren nach einem der Ansprüche 10-11, wobei das Nukleinsäuremolekül ein zirkulationsfreies DNA-Molekül ist.

## Revendications

1. Formulation destinée à un stockage substantiellement stable d'une ou de plusieurs molécule(s) d'acides nucléiques dans un état naturel, non dénaturé, dans un échantillon sanguin à des températures ambiantes, dans laquelle l'une ou les plusieurs molécule(s) d'acides nucléiques est/sont stabilisée(s) dans un état naturel, non dénaturé, substantiellement dépourvu d'acides nucléiques intracellulaires contaminateurs après un stockage à la température de la pièce pendant une période d'au moins quatre jours, la formulation comprenant :
(i) un tampon de pH ;
(ii) un inhibiteur de phosphatase ;
(iii) une purine ;
(iv) un composé zwittérionique, un composé cationique ou une combinaison de ceux-ci ;
et
(v) un composé choisi parmi :
(v.a) un inhibiteur d'apoptose ou un inhibiteur de caspase ; et/ou
(v.b) un inhibiteur de nucléase ;
et ladite formulation ne comprenant pas d'agent chélateur, tel que l'EDTA.

2. Formulation de la revendication 1, dans laquelle l'inhibiteur de phosphatase est le 2-glycérol phosphate.

3. Formulation des revendications 1 ou 2,
i) dans laquelle le composé zwittérionique est un sel interne quaternaire, le 2-(benzyl(2-hydroxyéthyl)(méthyl)ammonio)acétate, le propionate de N,N-diméthyl-N-(2-hydroxyéthyl)-3-ammonium ou le N-éthyl-pipéridinium-4-butylsulfonate ; ou un composé de formule (I) : 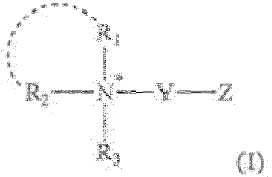 dans laquelle R1, R2 et R3 sont indépendamment choisis parmi un alkyle non substitué ou substitué, un aryle non substitué ou substitué, un arylalkyle non substitué ou substitué ou bien R1 et R2 forment, en option, un cycle, Y est CH₂, CH(A), CH(A)-CH(A), CH(A)-CH(A)-CH(A), dans lequel A est un alkyle, un aryle, un arylalkyle non substitué ou substitué ou bien une quelconque chaîne latérale que l'on trouve typiquement dans l'un des 20 acides aminés existant naturellement ; et Z est CO₂-, SO₃- ou OPO₃- ; ou
ii) dans laquelle le composé cationique est choisi dans le groupe constitué par :
(a) un composé de formule (II) : 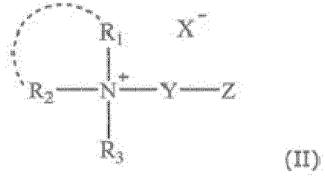 dans laquelle R1, R2 et R3 sont indépendamment choisis parmi un alkyle non substitué ou substitué, un aryle non substitué ou substitué, un arylalkyle non substitué ou substitué ou bien R1 et R2 forment, en option, un cycle, Y est CH₂, CH(A), CH(A)-CH(A), CH(A)-CH(A)-CH(A), où A est un alkyle, un aryle, un arylalkyle non substitué ou substitué ou bien une quelconque chaîne latérale que l'on trouve typiquement dans l'un des 20 acides aminés existant naturellement ; Z est CO₂A ; et X est un anion acceptable d'un point de vue pharmaceutique ;
(b) un composé de formule (III) : 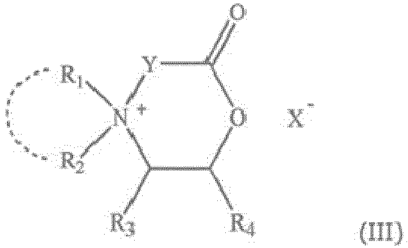 dans laquelle R1, R2, R3 et R4 sont indépendamment choisis parmi un alkyle non substitué ou substitué, un aryle non substitué ou substitué, un arylalkyle non substitué ou substitué ou bien R1 et R2 forment, en option, un cycle, Y est CH₂, CH(A), CH(A)-CH(A), CH(A)-CH(A)-CH(A), où A est un alkyle, un aryle, un arylalkyle non substitué ou substitué ou bien une quelconque chaîne latérale que l'on trouve typiquement dans l'un des 20 acides aminés existant naturellement ; et X est un anion acceptable d'un point de vue pharmaceutique ; et
(c) un composé de formule (IV) : 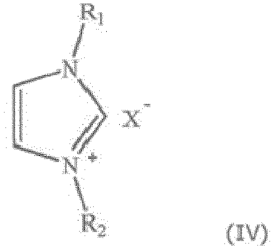 dans laquelle R1 et R2 sont indépendamment choisis parmi un alkyle non substitué ou substitué, un aryle non substitué ou substitué, un arylalkyle non substitué ou substitué ; et X est un anion acceptable d'un point de vue pharmaceutique.

4. Formulation de la revendication 3, dans laquelle les R1 et R2 d'un composé de formule (I), de formule (II) ou de formule (III) forment un cycle de morpholino, un cycle de pyrrolidinium, un cycle de pipéridinium ou un cycle d'oxazinium.

5. Formulation de l'une quelconque des revendications 1 à 4, dans laquelle
• l'inhibiteur de nucléase est un acide aurine tricarboxylique ;
• la purine est une adénine ; et/ou
• l'inhibiteur de caspase est Q-VD-OPH.

6. Formulation de l'une quelconque des revendications 1 à 5, comprenant en outre un agent choisi dans le groupe constitué par un antibiotique, un dérivé de purine et une combinaison de ceux-ci.

7. Formulation de la revendication 6, dans laquelle
• l'antibiotique est choisi dans le groupe constitué par la rifampicine, l'actinomycine D, la 5-hydroxy-1,4-naphtoquinone et une combinaison de ceux-ci ;
• le dérivé de purine est la 5-mercaptopurine.

8. Formulation de l'une quelconque des revendications 1 à 7, dans laquelle l'une ou les plusieurs molécule(s) d'acides nucléiques est/sont une molécule d'ADN non circulante.

9. Composition d'une ou de plusieurs molécule(s) d'acides nucléiques purifiée(s), non dénaturée(s), stockée(s) de manière substantiellement stable, comprenant une ou plusieurs molécule(s) d'acides nucléiques purifiée(s), non dénaturée(s) mélangée(s) avec une formulation de l'une quelconque des revendications 1 à 8.

10. Procédé destiné à un stockage substantiellement stable d'une ou de plusieurs molécule(s) d'acides nucléiques dans un état naturel, non dénaturé, dans un échantillon sanguin à des températures ambiantes, comprenant l'étape consistant à : mélanger un échantillon de sang prélevé à partir d'un sujet avec une formulation de l'une quelconque des revendications 1 à 8, dans lequel l'une ou les plusieurs molécule(s) d'acides nucléiques reste/restent dans un état naturel, non dénaturé, substantiellement dépourvu d'acides nucléiques intracellulaires contaminateurs après un stockage à la température de la pièce pendant une période d'au moins trois jours.

11. Procédé de la revendication 10, dans lequel la molécule d'acides nucléiques code pour un polypeptide choisi dans le groupe constitué par un anticorps, une enzyme et une protéine sérique, un anticorps, une enzyme, une protéine plasmatique et une protéine sérique.

12. Procédé de l'une quelconque des revendications 10-11, dans lequel la molécule d'acides nucléiques est une molécule d'ADN non circulante.
